(19) Europäisches Patentamt
European Patent Office
Office européen des brevets

(11) Veröffentlichungsnummer: **0 199 047**
**B1**

(12) # EUROPÄISCHE PATENTSCHRIFT

(45) Veröffentlichungstag der Patentschrift: **05.10.88**

(21) Anmeldenummer: **86103196.1**

(22) Anmeldetag: **10.03.86**

(51) Int. Cl.⁴: **C 07 D 317/22**, C 07 D 317/72, C 07 D 319/06, C 07 D 307/12, C 07 D 307/20, C 07 D 309/06, C 07 D 305/06, A 01 N 43/02, A 61 K 31/335

(54) **Neue Iodpropargylether, ein Verfahren zu ihrer Herstellung und ihre Verwendung.**

(30) Priorität: **21.03.85 DE 3510203**

(43) Veröffentlichungstag der Anmeldung: **29.10.86 Patentblatt 86/44**

(45) Bekanntmachung des Hinweises auf die Patenterteilung: **05.10.88 Patentblatt 88/40**

(84) Benannte Vertragsstaaten: **BE CH DE FR GB IT LI NL SE**

(56) Entgegenhaltungen:
**DE - A - 3 304 899**

**CHEMICAL ABSTRACTS, Band 101, Nr. 19, 5. November 1984, Seite 706, Zusammenfassung Nr. 171277d, Columbus, Ohio, US; & CS-A-211 195 (D. HESOUN et al.) 15-02-1984**
**J. ORG. CHEM., Band 47, Nr. 4, 12. Januar 1982, Seiten 725-730, American Chemical Society, US; R.J. SUNDBERG et al.: "Synthesis and intramolecular cycloaddition reactions of some 3-substituted 6-azidohexa-2,4-dienoate esters"**

(73) Patentinhaber: **BAYER AG, Konzernverwaltung RP Patentabteilung, D-5090 Leverkusen 1 Bayerwerk (DE)**

(72) Erfinder: **Schade, Gerold, Dr., Hahnenweg 8, D-5000 Köln 80 (DE)**
Erfinder: **Paulus, Wilfried, Dr., Deswatinesstrasse 90, D-4150 Krefeld (DE)**
Erfinder: **Schmitt, Hans-Georg, Dr., Am Oberend 13, D-4150 Krefeld (DE)**

**EP 0 199 047 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Iodpropargylether, ein Verfahren zu ihrer Herstellung und ihre Verwendung in mikrobiziden Mitteln.

Aus der DE-OS 3 304 899 ist bekannt, Iodpropargylether, wie 1-(3-Iod-2-propinyloxy)-propan-2,3--diol sowie davon abgeleitete Monoalkyl-alkenyl- und -phenylether, als antimikrobielle Substanzen zu verwenden. Nachteilig ist ihre nicht immer befriedigende Wirksamkeit.

Aus J. Org. Chem. 47, 729 (1982) ist, ohne Wirkungshinweis, die Verbindung 2-[3-Iodoprop-2--ynyl-oxy]tetrahydropyran bekannt.

Es wurden neue Iodpropargylether der Formel

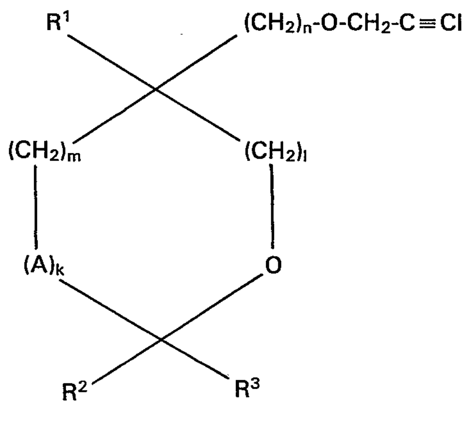

(I),

worin

A für Sauerstoff oder eine Methylengruppe steht,
$R^1$ Wasserstoff oder niederes Alkyl bedeutet,
$R^2$ und $R^3$ gleich oder verschieden sind und für Wasserstoff, niederes Alkyl, Alkenyl oder für gegebenenfalls durch Halogen substituiertes Phenyl stehen oder zusammen und unter Einschluss des sie tragenden C-Atoms einen carbocyclischen Ring mit 4 bis 7 Kohlenstoffatomes bilden,
l und m für 0, 1 oder 2 stehen,
n eine ganze Zahl von 0 bis 4 ist, mit der Massgabe, dass, wenn 1 = 0 ist, n für 1, 2, 3 oder 4 steht und
k 0 oder 1 bedeutet,
gefunden.

Erfindungsgemäss bedeutet niederes Alkyl allgemein einen geradkettigen oder verzweigten Kohlenwasserstoffrest mit 1 bis 6 Kohlenstoffatomen, bevorzugt 1 bis 4 Kohlenstoffatomen. Im einzelnen seien genannt: Methyl, Ethyl, Propyl, Isopropyl, Butyl, Isobutyl, Pentyl, Isopentyl, Hexyl und Isohexyl. Bevorzugt sind der Methyl- und der Ethylrest.

Halogen bedeutet erfindungsgemäss Fluor, Chlor, Brom und Iod, bevorzugt Chlor.

Falls $R^2$ und $R^3$ zusammen einen carbocyclischen Ring bilden, so ist ein carbocyclischer Ring mit 5 bis 6 Kohlenstoffatomen bevorzugt.

Bevorzugte neue Iodpropargylether sind solche der Formel

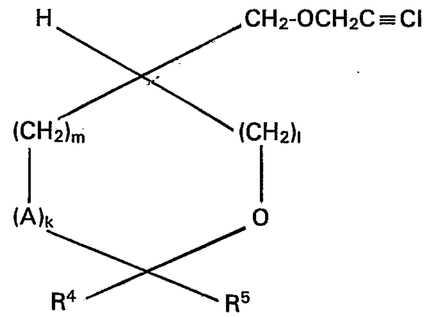

(II),

worin

A für Sauerstoff oder eine Methylengruppe steht,
$R^4$ Wasserstoff oder niederes Alkyl bedeutet,
$R^5$ für Wasserstoff, nieders Alkyl, Phenyl oder Chlorphenyl steht,
l und m für 0, 1 oder 2 stehen und
k 0 oder 1 bedeutet.

Beispielsweise seien die folgenden neuen Iodpropargylether genannt:
2-(4-Chlorphenyl)-5-iodpropargyloxy-1,3-dioxan,
2,2-Dimethyl-4-(4-iodpropargyloxybutyl)-1,3-dioxolan,
5-Ethyl-5-iodpropargyloxymethyl-1,3-dioxan,
2,2-Dimethyl-5-ethyl-5-iodpropargyloxymethyl--1,3-dioxan,
2,2,5-Trimethyl-5-iodpropargyloxymethyl-1,3--dioxan,
3-Iodpropargyloxy-tetrahydrofuran,
3-Methyl-3-iodpropargyloxymethyl-oxetan,
3-Ethyl-3-iodpropargyloxymethyl-oxetan,
4-Iodpropargyloxymethyl-1,3-dioxolan,
2-Methyl-4-(iodpropargyloxymethyl)-1,3-dioxolan,
2,2-Dimethyl-4-(iodpropargyloxymethyl)-1,3--dioxolan,
2-Methyl-2-ethyl-4-(iodpropargyloxymethyl)-1,3--dioxolan,
2,2-Pentamethylen-4-(iodpropargyloxymethyl)--1,3-dioxolan,
2-Phenyl-4-(iodpropargyloxymethyl)-1,3-dioxolan,
2-Iodpropargyloxymethyl-tetrahydrofuran,
2-Iodpropargyloxymethyl-tetrahydropyran und
2-Methyl-2-phenyl-4-(iodpropargyloxymethyl)-1,3--dioxolan,

bevorzugt
4-(Iodpropargyloxymethyl-1,3-dioxolan,
2-Methyl-4-(iodpropargyloxymethyl)-1,3-dioxolan,
2,2-Dimethyl-4-(iodpropargyloxymethyl)-1,3--dioxolan,
2-Methyl-2-ethyl-4-(iodpropargyloxymethyl)-1,3--dioxolan,
2,2-Pentamethylen-4-(iodpropargyloxymethyl)--1,3-dioxolan,
2-Phenyl-4-(iodpropargyloxymethyl)-1,3-dioxolan,
2-Iodpropargyloxymethyl-tetrahydrofuran,
2-Iodpropargyloxymethyl-tetrahydropyran und
2-Methyl-2-phenyl-4-(iodpropargyloxymethyl)-1,3--dioxolan.

Weiterhin wurde ein Verfahren zur Herstellung der neuen Iodpropargylether der Formel

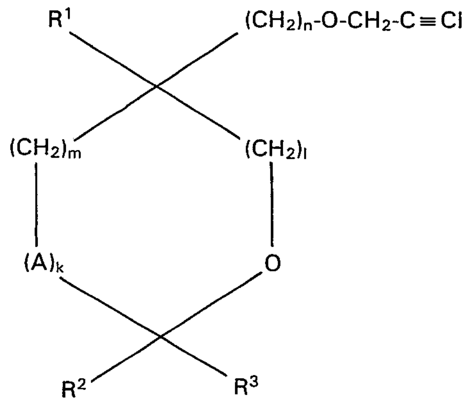

(I),

worin

A für Sauerstoff oder eine Methylengruppe steht,
$R^1$ Wasserstoff oder niederes Alkyl bedeutet,
$R^2$ und $R^3$ gleich oder verschieden sind und für Wasserstoff, niederes Alkyl, Alkenyl oder für gegebenenfalls durch Halogen substituiertes Phenyl stehen oder zusammen einen carbocyclischen Ring mit 4 bis 7 Kohlenstoffatomen bilden,
l und m für 0, 1 oder 2 stehen,
n eine ganze Zahl von 0 bis 4 ist, mit der Massgabe, dass, wenn 1 = 0 ist, n für 1, 2, 3 oder 4 steht und
k 0 oder 1 bedeutet,

gefunden, das dadurch gekennzeichnet ist, dass man Propargylether der Formel

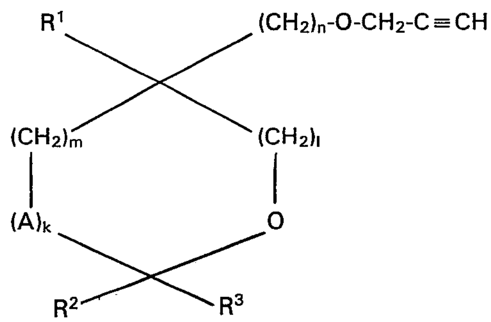

(III),

worin

A, $R^1$, $R^2$, $R^3$, l, m, n und k die obengenannte Bedeutung besitzen,

mit Iodierungsmitteln in Gegenwart von Lösungs- und/oder Verdünnungsmitteln und in Gegenwart von Basen bei Temperaturen von −10 bis 30°C umsetzt.

Als Iodierungsmittel können in das erfindungsgemässe Verfahren Iod und/oder Iodidionen liefernde Verbindungen, wie Natriumiodid und Ammoniumiodid, in Gegenwart von Oxidationsmitteln, wie Natriumhypochlorit, Calciumhypochlorit und Wasserstoffperoxid, eingesetzt werden.

Als Basen eignen sich sowohl anorganische als auch organische Basen, wie Natriumhydroxid, Calciumhydroxid, Natriummethylat, Kalium-tert. butylat und Natriumisobutylat, bevorzugt Natriumhydroxid und Natriummethylat.

Geeignete Lösungsmittel für das erfindungsgemässe Verfahren sind beispielsweise Wasser oder Alkohole, wie Methanol und/oder Ethanol, oder Gemische derselben.

Bevorzugt wird die Iodierung bei Temperaturen von −5°C bis +20°C durchgeführt.

Erfindungsgemäss setzt man 1 Mol Propargylether der allgemeinen Formel (III) mit etwa 1 bis 1,5 Mol Iodierungsmittel, bevorzugt 1 bis 1,2 Mol Iodierungsmittel um.

Die jeweils günstigsten Mengen an Basen und an Lösungs- und/oder Verdünnungsmittel lassen sich leicht durch Vorversuche ermitteln. Üblicherweise setzt man etwa 1 bis 3, bevorzugt 1,5 bis 2, Mol Base pro Mol Propargylether der allgemeinen Formel (III) und die gleiche bis fünffache, bevorzugt die doppelte bis dreifache, Gewichtsmenge an Lösungs- und/oder Verdünnungsmittel ein.

Die zur Herstellung der neuen Iodproparghylether der Formel (I) einzusetzenden Propargylether der allgemeinen Formel (III) sind zum Teil bekannt (vgl. US-PS 3 290 388). Sie können analog zu den dort beschriebenen Verfahren hergestellt werden, indem man die entsprechenden Hydroxyverbindungen der allgemeinen Formel

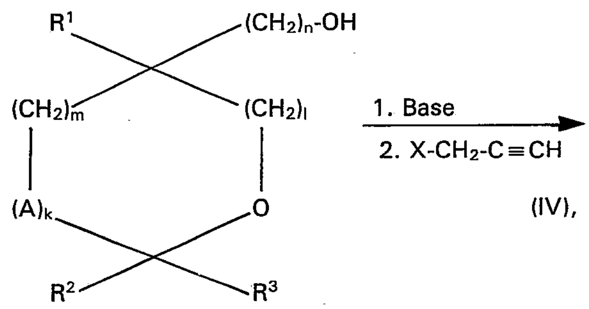


(IV),

worin

A Für Sauerstoff oder eine Methylengruppe steht,
$R^1$ Wasserstoff oder niederes Alkyl bedeutet,
$R^2$ und $R^3$ gleich oder verschieden sind und für Wasserstoff, niederes Alkyl, Alkenyl oder für gegebenenfalls durch Halogen substituiertes Phenyl stehen oder zusammen einen carbocyclischen Ring mit 4 bis 7 Kohlenstoffatomen bilden,
l und m für 0, 1 oder 2 stehen,
n eine ganze Zahl von 0 bis 4 ist, mit der Massgabe, dass, wenn 1 = 0 ist, n für 1, 2, 3 oder 4 steht und
k 0 oder 1 bedeutet,

mit Propargylhalogeniden in Gegenwart von Basen und in Gegenwart von Lösungs- und/oder Verdünnungsmitteln bei Temperaturen von etwa 0 bis 100°C umsetzt.

Als Basen eignen sich für die Umsetzung besonders starke Basen, wie Natriumhydrid, Natriumamid und/oder Kaliumtert.-butylat.

Als Propargylhalogenide seien genannt: Propargylchlorid und Propargylbromid, bevorzugt Propargylchlorid.

Als Lösungsmittel können eingesetzt werden solche Lösungsmittel, die gegen die eingesetzten Basen inert sind; beispielsweise kommen Dimethylformamid, Tetrahydrofuran, Dimethoxyethan und/oder Toluol in Frage.

Die Umsetzung der Hydroxyverbindungen der Formel (IV) wird zweckmässigerweise so durchgeführt, dass man zunächst die Deprotonierung mit der Base durchführt, und zwar in der Weise, dass man zuerst bei niedrigen Reaktionstemperaturen arbeitet (ca. 0 bis 20 °C) und dann die Reaktionen durch Erwärmen auf Temperaturen von etwa 20 bis 60 °C zu Ende führt.

Nach erfolgter Deprotonierung wird das entsprechende Propargylhalogenid zugesetzt. Die zur Etherbildung erforderliche Reaktionstemperatur hängt im allgemeinen von der Reaktivität des Alkoholats der Verbindung (IV) ab und liegt im allgemeinen bei etwa 20 bis 100 °C, bevorzugt 20 bis 60 °C. Sollte es erforderlich werden, kann die Temperatur noch während der Umsetzung gesteuert werden.

Weiterhin kann es von Vorteil sein, die Umsetzung der Hydroxyverbindung der Formel (IV) mit einer Base und einem Propargylhalogenid im wässrig-organischen Zweiphasensystem unter Phasentransferkatalyse durchzuführen. Es ist dann möglich Natriumhydroxid als Base einzusetzen. Geeignete organische Lösungsmittel für die Phasentransferreaktion sind beispielsweise Dichlormethan, Tetrahydrofuran und/oder Toluol. Als Phasentransferkatalysatoren können die bekannten Tetraalkylammoniumsalze, wie Triethylbenzylammoniumchlorid, Tetrabutylammoniumbromid und Dimethyldodecylbenzylammoniumchlorid, oder Kronenether, wie 18-Krone-6 und Dibenzo-18-Krone-6, eingesetzt werden (vgl. Dehmlow u. Dehmlow, Phase Transfer Catalysis, Weinheim 1983).

Die einzusetzende Menge an Basen, Hydroxyverbindungen der Formel (IV) und Propargylhalogeniden kann wiederum leicht durch Vorversuche ermittelt werden.

Die Hydroxyverbindungen der allgemeinen Formel (IV) sind teilweise literaturbekannt. Wenn A gleich Sauerstoff und k = 1 ist, können sie analog dem in Organic Syntheses Coll. Vol. 3, Seite 502 beschriebenen Verfahren hergestellt werden, indem man Trihydroxyverbindungen der Formel

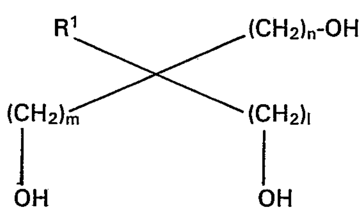 (V),

worin

$R^1$, l, m und n die obengenannte Bedeutung besitzen,

mit Carbonylverbindungen der Formel

$R^2$-CO-$R^3$ (VI),

worin

$R^2$ und $R^3$ die obengenannte Bedeutung haben,

unter Wasserabspaltung kondensiert.

Setzt man beispielsweise Glycerin mit Carbonylverbindungen um, so können je nach Art der Carbonylverbindung Dioxolane (VII) oder Dioxane /VIII) entstehen (siehe nachfolgendes Formelschema).

HO-$CH_2$-CH(OH)-$CH_2$-OH + $R^2$-CO-$R^3$

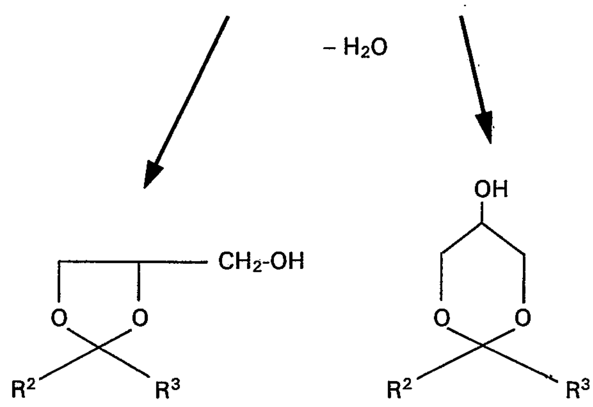


(VII) (VIII)

Bei dieser Umsetzung erhält man gelegentlich Gemische, aus denen die reinen Verbindungen (VII) und (VIII) durch bekannte Trennmethoden, z.B. Destillation oder Chromatographie, isoliert werden können. Es kann auch zweckmässig sein, das Gemisch in die weiteren Syntheseschritte einzusetzen, wodurch man schliesslich ein Gemisch erfindungsgemässer, neuer Iodpropargylether erhält.

Die erfindungsgemässen Iodpropargylether der Formel

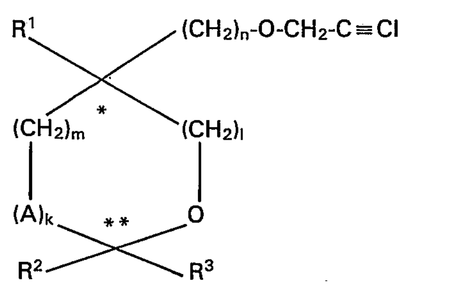 (I)

können Stereoisomere bilden. So ist das mit einem Stern gekennzeichnete Kohlenstoffatom chiral, wenn l ungleich m ist oder A ungleich Sauerstoff ist. Wenn in diesen Fällen zusätzlich $R^2$ ungleich $R^3$ ist, ist auch das mit zwei Sternen gekennzeichnete Kohlenstoffatom chiral. Doch auch wenn die gekennzeichneten Kohlenstoffatome nicht chiral sind, können cis/trans-Isomere bezüglich des Ringsystems gebildet werden; nämlich dann, wenn $R^2$ ungleich $R^3$ ist.

Die möglichen Enantiomere, Diastereomere und cis/trans-Isomere der erfindungsgemässen Iodpropargylether können durch bekannte Methoden getrennt werden, beispielsweise durch Kristallisation, Destillation oder Umsetzung mit chiralen Hilfsreagentien (vgl. E. Eliel, Stereochemie der Kohlenstoffverbindungen, Weinheim 1966).

Oft dürfte es aber zweckmässig sein, auf die Trennung zu verzichten und die Isomerengemische zu verwenden.

Die Erfindung umfasst sowohl die reinen Isomeren als auch deren Gemische.

Die neuen erfindungsgemässen Iodpropargylether können als Wirkstoffe zur Bekämpfung von Mikroorganismen, im besonderen zum Schutz von technischen Materialien, verwendet werden.

Technische Materialien sind erfindungsgemäss nicht lebende Materialien, die für die Verwendung in der Technik zubereitet worden sind. Beispielsweise können technische Materialien, die durch erfindungsgemässe Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemässen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeroginosa,
Staphylococcus, wie Staphylococcus aureus.

Je nach Anwendungsgebiet kann ein erfindungsgemässer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95%, bevorzugt von 10 bis 75%.

Die Anwendungskonzentrationen der erfindungsgemässen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentration im Bereich von 0,001 bis 5 Gew.-%, vorzugsweise von 0,05 bis 1,0 Gew.-%, bezogen auf das zu schützende Material.

Die erfindungsgemässen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol.

*Herstellungsbeispiele*

A) Herstellung von Dioxolanen und Dioxanen

1 Mol der entsprechenden Trihydroxyverbindung, 1 bis 4 Mol der entsprechenden Carbonylverbindung, 300 ml Petrolether und 3 g p-Toluolsulfonsäure wurden am Wasserabscheider bis zur Beendigung der Wasserbildung erhitzt. Man setzte 3 g Natriumacetat zu, rührte 30 Minuten lang, filtrierte, engte ein und destillierte.

So wurden erhalten:

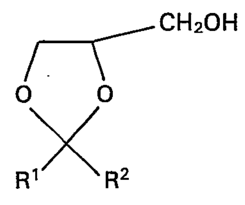

| Beispiel Nr. | $R^1$ | $R^2$ | Kp(°C)/Druck (mbar) | Ausbeute | Bemerkungen |
|---|---|---|---|---|---|
| 1 | $CH_3$ | $CH_3$ | 80/20 | 84% | |
| 2 | $CH_3$ | $C_2H_5$ | 93/20 | 81% | |
| 3 | $-(CH_2)_5-$ | | 97/0,4 | 71% | |
| 4 | $C_6H_5$ | $CH_3$ | 140/1,5 | 81% | |
| 5 | $4\text{-Cl-}C_6H_4$ | $CH_3$ | 150/0,9 | 34% | |
| 6 | H | $CH_3$ | 64/2,5 | 53% | x) |
| 7 | H | $C_6H_5$ | 120/1,2 | 62% | x) |
| 8 | H | $4\text{-Cl-}C_6H_4$ | Schmp. 110°C | 23% | x) |

x) Gemisch mit dem entsprechenden Dioxan 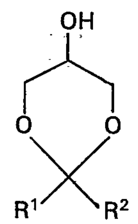

| Beispiel Nr. | | | Kp/Druck | Ausbeute |
|---|---|---|---|---|
| 9 | 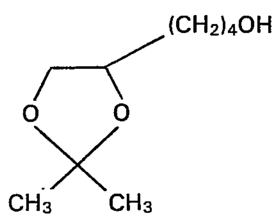 | | 121/20 | 45% |
| 10 | 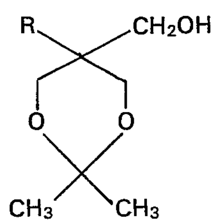 | R = $CH_3$ | 110/8 | 84% |
| 11 | | R = $C_2H_5$ | 112/0,35 | 76% |

B) Herstellung von Propargylethern

a) 1,05 Mol Natriumhydrid wurde in Dimethylformamid suspendiert und 1 Mol Hydroxyverbindung zugetropft. Man rührte bis zur Beendigung der Wasserstoffentwicklung nach. Dann tropfte man 1,1 Mol Chlorpropin zu und rührte nach, bis die Reaktion vollständig abgelaufen war. Man filtrierte, engte ein und destillierte.

So wurden erhalten:

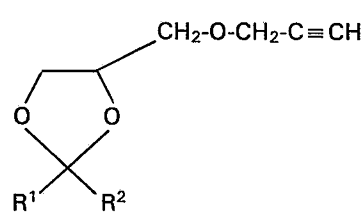

| Beispiel Nr. | $R^1$ | $R^2$ | Kp/Druck | Ausbeute | Bemerkung |
|---|---|---|---|---|---|
| 12 | $CH_3$ | $CH_3$ | 83/3,3 | 44% | |
| 13 | $CH_3$ | $C_2H_5$ | 67/0,7 | 55% | |
| 14 | $-(CH_2)_5-$ | | 103/1,2 | 70% | |
| 15 | $C_6H_5$ | $CH_3$ | 120/1,5 | 25% | |
| 16 | 4-Cl-$C_6H_4$ | $CH_3$ | 132/0,6 | 35% | |
| 17 | H | $CH_3$ | 40-60/1,0 | 43% | x) |
| 18 | H | $C_6H_5$ | 119/0,8 | 29% | x) |
| 19 | 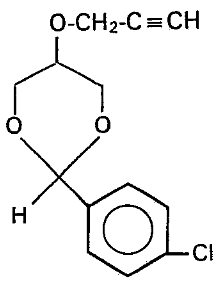 | | Schmp. 102°C | 44% | |

x) Gemisch mit dem entsprechenden Dioxan 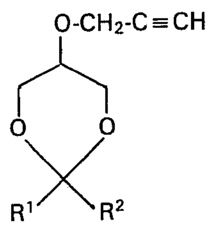

| Beispiel Nr. | | Kp/Druck | Ausbeute |
|---|---|---|---|
| 20 | 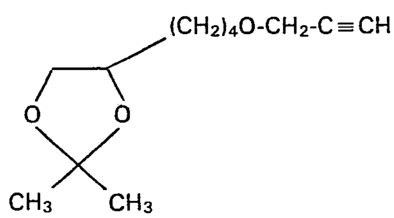 | 93/0,6 | 61% |
| 21 | 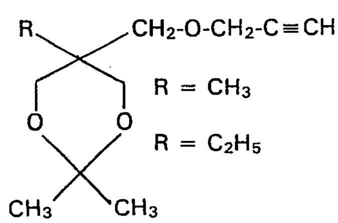 R = $CH_3$ | 85/2 | 47% |
| 22 | R = $C_2H_5$ | 82/1 | 38% |
| 23 | 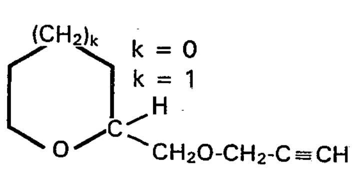 k = 0 | 57/0,5 | 16% |
| 24 | k = 1 | 70/4 | 34% |

| Beispiel Nr. | | Kp/Druck | Ausbeute |
|---|---|---|---|
| 25 | 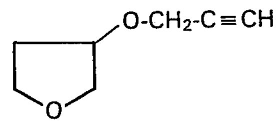 | 74/18 | 21% |
| 26 | 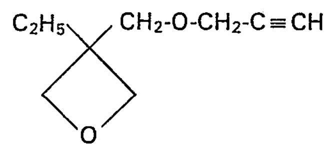 | 92/3,5 | 17% |

b) 0,5 Mol Hydroxyverbindung, 0,55 Mol Chlorpropin, 0,025 Mol Tetrabutylammoniumbromid, 150 ml Toluol und 250 ml 50% NaOH wurden zunächst 1 h bei 20°C und dann 1,5 h bei 60°C gerührt. Die organische Phase wurde abgetrennt und eingeengt. Der Rückstand konnte ohne weitere Reinigung in die Iodierung eingesetzt werden.

So wurden erhalten:

| Beispiel | Struktur | Ausbeute |
|---|---|---|
| 26a | 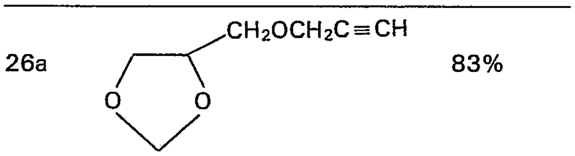 | 83% |
| 26b | 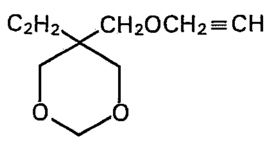 | 85% |
| 26c | 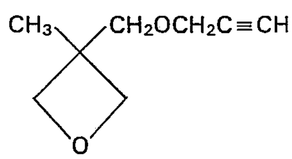 | 56% |

C) Herstellung von Iodpropargylethern

1 Mol Propargylether wurde in Methanol gelöst und auf 0°C gekühlt. Man gab 1,5 Mol wässrige Natronlauge und 1,1 Mol Iod hinzu und rührte bei 0 bis 5°C nach. Nach beendeter Iodierung engte man ein, versetzte das Reaktionsgemisch mit Wasser, entfärbte mit Natriumthiosulfat, nahm in Dichlormethan auf und engte ein. Die Produkte wurden im allgemeinen als Öl isoliert, in einigen Fällen trat Kristallisation ein.

So wurden erhalten:

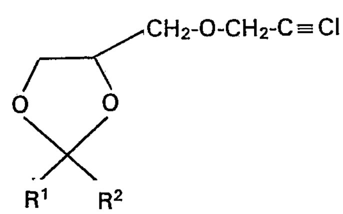

($^1$H-NMR-Daten: Lösungsmittel $CDCl_3$; TMS als innerer Standard)

| Beispiel Nr. | $R^1$ | $R^2$ | $^1$H-NMR, δ = | Ausbeute | Bemerkung |
|---|---|---|---|---|---|
| 27 | $CH_3$ | $CH_3$ | 1,35 (s, 3H); 1,42 (s, 3H); 3,5-4,3 (m, 5H); 4,35 (s, 2H); | 91% | |
| 28 | $CH_3$ | $C_2H_5$ | 0,9 (m, 3H); 1,29/1,34 (2s, 3H); 1,7 (m, 2H); 3,5-4,3 (m, 5H); 4,33 (s, 2H); | 86% | cis/trans |
| 29 | $-(CH_2)_5-$ | | 1,2-1,8 (m, 10H); 3,5-4,3 (m, 5H); 4,32 (s, 2H); | 80% | |
| 30 | $C_6H_5$ | $CH_3$ | 1,64/1,67 (2s, 3H); 3,3-4,2 (m, 5H); 4,25/4,37 (2s, 2H); 7,2-7,5 (m, 5H); | 99% | cis/trans |
| 31 | 4-Cl-$C_6H_4$ | $CH_3$ | 1,60/1,64 (2s, 3H); 3,3-4,2 (m, 5H); 4,25/4,37 (2s, 2H); 7,2-7,4 (m, 4H); | 100% | cis/trans |

| Beispiel Nr. | $R^1$ | $R^2$ | $^1$H-NMR, δ = | Ausbeute | Bemerkung |
|---|---|---|---|---|---|
| 32 | H | $CH_3$ | 1,3-1,4 (m, 3H); 3,5-4,4 (m, 5H); 4,34/4,46 (2s, 2H); 4,55/4,70/5,99/ 5,10 (4q, 1H) | 86% | x) |
| 33 | H | $C_6H_5$ | 3,6-4,5 (m, 5H); 4,35/4,38 (2s, 2H); 5,53/5,80/5,82/5,93 (4s, 1H); | 99% | x) |
| 33a | H | H | 3,3-4,3 (m, 5H); 4,34/4,37 (2s, 2H); 4,70/4,84 (AB); 4,86/5,00 (AB) (zus. 2H) | 46% | x) |

x) Gemisch mit dem entsprechenden Dioxan 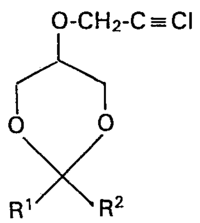 (evtl. jeweils cis/trans)

| Beispiel Nr. | | $^1$H-NMR, δ = | Ausbeute |
|---|---|---|---|
| 34 | 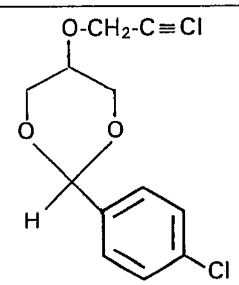 | 3,58 (s, 1H); 4,03/4,30 (AB, 4H); 4,45 (s, 2H); 5,46 (s, 1H); 7,27-7,4 (m, 4H) | 39% Schmp. 131 °C |
| 35 | 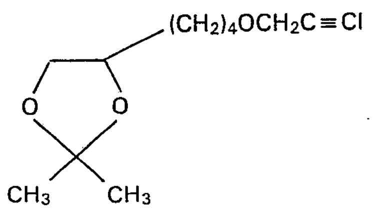 | 1,33 (s, 3H); 1,38 (s, 3H); 1,4-1,7 (m, 6H); 3,48 (t, 2H); 3,9-4,1 (m, 3H); 4,24 (m, 2H) | 94% |
| | | 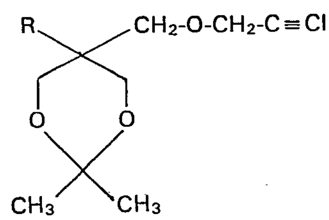 | |
| 36 | R = $CH_3$ | 0,86 (s, 3H); 1,37 (s, 3H); 1,41 (s, 3H); 3,5-3,8 (m, 6H); 4,28 (s, 2H) | 86% |
| 37 | R = $C_2H_5$ | 0,85 (t, 3H); 1,36 (q, 2H); 1,41 (s, 6H); 3,5-3,7 (m, 6H); 4,3 (s, 2H) | 90% Schmp. 65 °C |
| 37a | 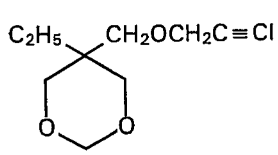 | 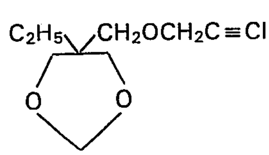 0,83 (t, 3H); 1,33 (q, 2H); 3,44/3,81 (AB, 4H); 3,56 (s, 2H); 4,25 (s, 2H); 4,62/4,87 (AB, 2H) | 75% Schmp. 45 °C |

| Beispiel Nr. | | $^1H$-NMR, δ = | Ausbeute |
|---|---|---|---|
| | | 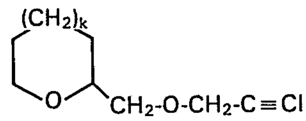 | |
| 38 | K = 0 | 1,6-2,0 (m, 4H); 3,4-4,1 (m, 5H); 4,34 (s, 2H) | 81% |
| 39 | K = 1 | 1,2-1,9 (m, 6H); 3,4-4,0 (m, 5H); 4,30 (s, 2H) | 99% |
| 40 | 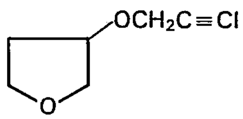 | 1,9-2,1 (m, 2H); 3,7-3,9 (m, 4H); 4,26 (s, 2H); 4,29 (m, 1H) | 78% |
| 41 | 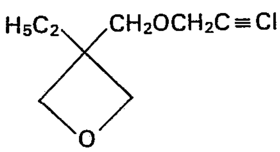 | 0,9 (t, 3H); 1,75 (q, 2H); 3,65 (s, 2H); 4,33 (s, 2H); 4,3-4,5 (m, 4H) | 86% |
| 42 | 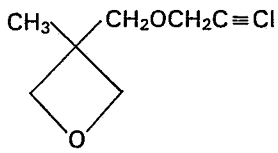 | 1,30 (s, 3H); 3,55 (s, 2H); 4,31 (s, 2H); 4,33/4,48 (AB, 4H) | 85% |

*Anwendungsbeispiele*

Als Vergleichssubstanz dient 1-(Iodpropargyloxy)-propan-2,3-diol (DE-OS 3 304 899).

*Beispiel 1*

Zum Nachweis der Wirksamkeit gegen Pilze werden die minimalen Hemm-Konzentrationen (MHK) von erfindungsgemässen Wirkstoffen bestimmt:

Ein Agar, der aus Bierwürze und Pepton hergestellt wird, wird mit erfindungsgemässen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70% rel. Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

TABELLE I

Minimale Hemmkonzentrationen (mg/l) erfindungsgemässer Substanzen für Pilze

Verbindungen

| Testorganismen Bsp. Nr. | 27 | 29 | 28 | 32 | 33 | 30 | 35 | 38 | 39 | 37 | 36 | 34 | 41 | Vergleich |
|---|---|---|---|---|---|---|---|---|---|---|---|---|---|---|
| Penicillium glaucum | 15 | 15 | 15 | 10 | 20 | 50 | 50 | 5 | 5 | 100 | 50 | 10 | 5 | 50 |
| Chaetomium globosum | 10 | 3,5 | 10 | 10 | 7,5 | 3,5 | 7,5 | 10 | 5 | 15 | 20 | 3,5 | 5 | 100 |
| Aspergillus niger | 5 | 5 | 5 | 7,5 | 7,5 | 10 | 5 | 3,5 | 5 | 10 | 10 | 5 | 5 | 50 |

TABELLE I (Fortsetzung)

Minimale Hemmkonzentrationen (mg/l) erfindungsgemässer Substanzen für Pilze

| Testorganismen | Bsp. Nr. | 27 | 29 | 28 | 32 | 33 | 30 | 35 | 38 | 39 | 37 | 36 | 34 | 41 | Vergleich |
|---|---|---|---|---|---|---|---|---|---|---|---|---|---|---|---|
| | | Verbindungen | | | | | | | | | | | | | |
| Trichoderma viride | | 100 | 200 | 100 | 50 | 100 | 750 | 500 | 75 | 50 | 500 | 200 | 1000 | 100 | 500 |
| Alternaria tenuis | | 10 | 50 | 35 | 5 | 50 | 50 | 100 | 5 | 5 | 100 | 50 | 15 | 15 | 50 |
| Aureobasidium pullulans | | 20 | 35 | 20 | 10 | 35 | 200 | 100 | 5 | 10 | 150 | 75 | 20 | 20 | 50 |
| Sclerophoma pityophila | | 20 | 20 | 20 | 5 | 10 | 50 | 50 | 5 | 5 | 75 | 50 | 15 | 35 | 50 |
| Lentinus tigrinus | | 10 | 20 | 20 | 75 | 10 | 50 | 50 | 10 | 5 | 100 | 10 | 1000 | 10 | 50 |

*Beispiel 2*

Wirkung gegen Bakterien

Ein Agar, der als Nährmedium Bouillon enthält, wird mit erfindungsgemässen Wirkstoffen in Konzentrationen von 1 bis 5000 ppm versetzt. Darauf infiziert man das Nährmedium jeweils mit den in Tabelle II aufgeführten Testorganismen und hält das infizierte Medium 2 Wochen bei 28°C und 60 bis 70% rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle II wiedergegeben.

TABELLE II

Angabe der MHK-Werte in mg/l bei der Einwirkung der unten angegebenen Wirkstoffe auf Bakterien

| Testorganismen | Bsp. Nr. | 27 | 28 | 32 | 35 | 38 | 39 | 36 | 41 | Vergleich |
|---|---|---|---|---|---|---|---|---|---|---|
| | | MHK in mg/l der Wirkstoffe | | | | | | | | |
| Escherichia coli | | 500 | 500 | 200 | 750 | 200 | 500 | 750 | 500 | 200 |
| Staphylococcus aureus | | 200 | 100 | 500 | 100 | 100 | 100 | 100 | 200 | 200 |

*Beispiel 3*

(Wirkung gegen Schleimorganismen)

Erfindungsgemässe Susbtanzen werden in Konzentrationen von jeweils 0,1 bis 100 mg/l in Allens Nährlösung [Arch. Mikrobiol. 17, 34 bis 53 (1952)], die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder grössere Wirkstoffkonzentrationen aufweisen, sind nach 2-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

TABELLE III

MHK-Werte in mg/l bei der Einwirkung der unten angegebenen Substanzen auf Schleimorganismen

| Wirkstoff | MHK in mg/l |
|---|---|
| Beispiele 27 | 25 |
| 29 | 15 |
| 28 | 25 |
| 35 | 25 |
| 38 | 25 |
| 39 | 25 |
| 37 | 25 |
| 36 | 25 |
| Vergleich | 35 |

*Beispiel 4*

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung [Arch. Mikrobiol. 17, 34 bis 53 (1952)], die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemässer Wirkstoffe erkennt man an dem Entfärben der Nährlösung.

TABELLE IV

Algen-abtötende Konzentration (mg/l) der unten angegeben Substanzen

| Wirkstoff | abtötende Konzentration in mg/l |
|---|---|
| Beispiel 38 | 100 |
| Beispiel 39 | 100 |
| Beispiel 27 | 100 |
| Vergleich | > 100 |

**Patentansprüche**

1. Iodpropargylether der Formel

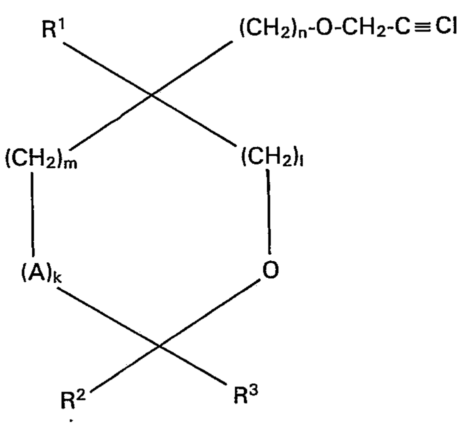

,

worin

A für Sauerstoff oder eine Methylengruppe steht,

$R^1$ Wasserstoff oder $C_1$-$C_4$-Alkyl bedeutet,

$R^2$ und $R^3$ gleich oder verschieden sind und für Wasserstoff, $C_1$-$C_6$-Alkyl, Alkenyl oder für gegebenenfalls durch Halogen substituiertes Phenyl stehen oder zusammen und unter Einschluss des sie tragenden C-Atoms einen carbocyclischen Ring mit 4 bis 7 Kohlenstoffatomen bilden,

l und m für 0, 1 oder 2 stehen,

n eine ganze Zahl von 0 bis 4 bedeutet, mit der Massgabe, dass, wenn 1 = 0 ist, n für 1, 2, 3 und 4 steht und

k 0 oder 1 bedeutet.

2. Verfahren zur Herstellung der neuen Iodpropargylether der Formel

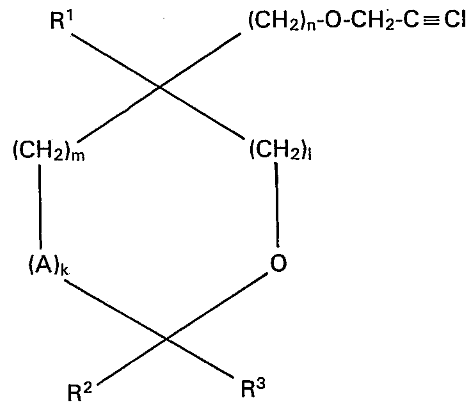

,

worin

A für Sauerstoff oder eine Methylengruppe steht,

$R^1$ Wasserstoff oder $C_1$-$C_6$-Alkyl bedeutet,

$R^2$ und $R^3$ gleich oder verschieden sind und für Wasserstoff, $C_1$-$C_6$-Alkyl, Alkenyl oder für gegebenenfalls durch Halogen substituiertes Phenyl stehen oder zusammen und unter Einschluss des sie tragenden C-Atoms einen carbocyclischen Ring mit 4 bis 7 Kohlenstoffatomen bilden,

l und m für 0, 1 oder 2 stehen,

n eine ganze Zahl von 0 bis 4 bedeutet, mit der Massgabe, dass, wenn 1 = 0 ist, n für 1, 2, 3 und 4 steht und

k 0 oder 1 bedeutet,

dadurch gekennzeichnet, dass man Propargylether der Formel

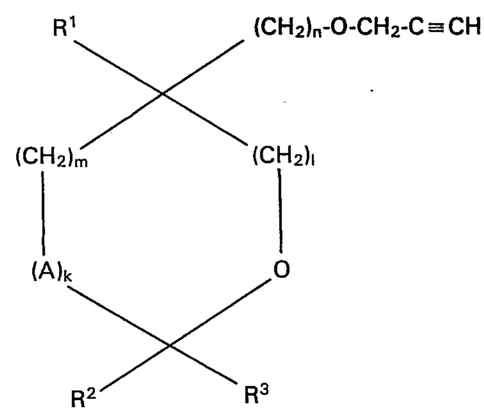

worin

A, $R^1$, $R^2$, $R^3$, l, m, n und k die obengenannte Bedeutung besitzen,

mit Iodierungsmittelm in Gegenwart von Lösungs- und/oder Verdünnungsmitteln und in Gegenwart von Basen bei Temperaturen von −10 bis 30°C umsetzt.

3. Mikrobizides Mittel, enthaltend die neuen Iodpropargylether der Formel

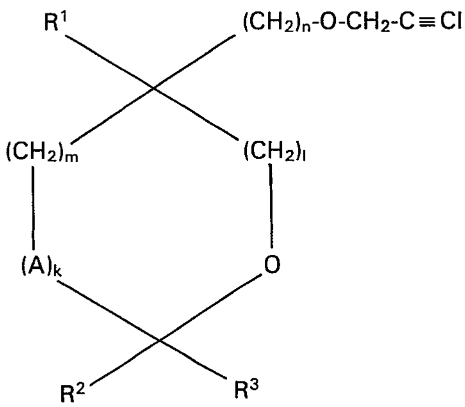

worin

A für Sauerstoff oder eine Methylengruppe steht,

$R^1$ Wasserstoff oder $C_1$-$C_6$-Alkyl bedeutet,

$R^2$ und $R^3$ gleich oder verschieden sind und für Wasserstoff, $C_1$-$C_6$-Alkyl, Alkenyl oder für gegebenenfalls durch Halogen substituiertes Phenyl stehen oder zusammen und unter Einschluss des sie tragenden C-Atoms einen carbocyclischen Ring mit 4 bis 7 Kohlenstoffatomen bilden,

l und m für 0, 1 oder 2 stehen,

n eine ganze Zahl von 0 bis 4 bedeutet, mit der Massgabe, dass, wenn 1 = 0 ist, n für 1, 2, 3 und 4 steht und

k 0 oder 1 bedeutet.

4. Mikrobizides Mittel nach Anspruch 3, enthaltend 1 bis 95 Gew.-% des neuen Iodpropargylethers.

5. Verwendung von mikrobiziden Mitteln nach den Ansprüchen 3 und 4 zum Schutz technischer Materialien.

6. Verwendung nach Anspruch 5 zum Schutz von Holz gegen holzverfärbende und holzzerstörende Pilze.

## Claims

1. Iodopropargyl ethers of the formula

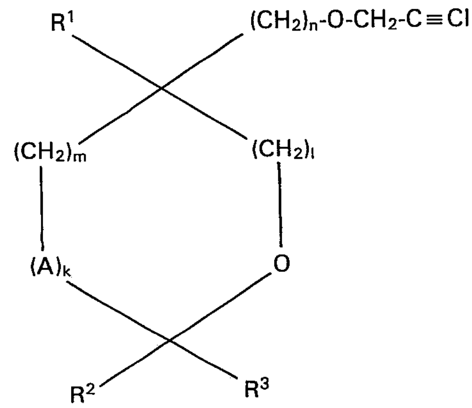

wherein

A represents oxygen or a methylene group,

$R^1$ denotes hydrogen or $C_1$-$C_6$-alkyl,

$R^2$ and $R^3$ are identical or different and represent hydrogen, $C_1$-$C_6$-alkyl, alkenyl or optionally halogen- substituted phenyl, or together, including the C atom carrying them, form a carbocyclic ring with 4 to 7 carbon atoms,

l and m represent 0, 1 or 2,

n denotes an integer from 0 to 4, with the proviso that if l is 0, n represents 1, 2, 3 or 4, and

k denotes 0 or 1.

2. Process for the preparation of the new iodopropargyl ethers of the formula

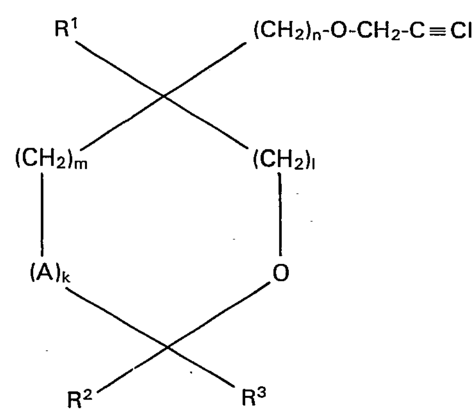

wherein

A represents oxygen or a methylene group,

$R^1$ denotes hydrogen or $C_1$-$C_6$-alkyl,

$R^2$ and $R^3$ are identical or different and represent hydrogen, $C_1$-$C_6$-alkyl, alkenyl or optionally halogen-substituted phenyl, or together, including the C atom carrying them, form a carbocyclic ring with 4 to 7 carbon atoms,

l and m represent 0, 1 or 2,

n denotes an integer from 0 to 4, with the proviso that if l is 0, n represents 1, 2, 3 or 4, and

k denotes 0 or 1,

characterised in that propargyl ethers of the formula

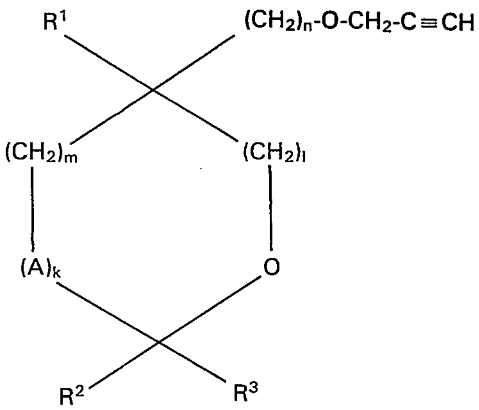

wherein

A, $R^1$, $R^2$, $R^3$, l, m, n and k have the abovementioned meaning,

are reacted with iodinating agents in the presence of solvents and/or diluents and in the presence of bases at temperatures of −10 to 30°C.

3. Microbicidal agents containing the new iodopropargyl ethers of the formula

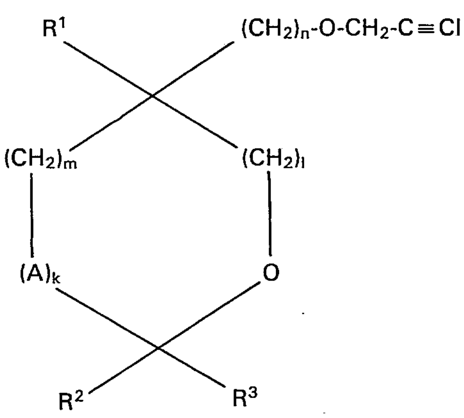

wherein

A represents oxygen or a methylene group,

$R^1$ denotes hydrogen or $C_1$-$C_6$-alkyl,

$R^2$ and $R^3$ are identical or different and represent hydrogen, $C_1$-$C_6$-alkyl, alkenyl or optionally halogen-substituted phenyl, or together, including the C atom carrying them, form a carbocyclic ring with 4 to 7 carbon atoms,

l and m represent 0, 1 or 2,

n denotes an integer from 0 to 4, with the proviso that if l is 0, n represents 1, 2, 3 or 4, and

k denotes 0 or 1.

4. Microbicidal agent according to Claim 3, containing 1 to 95% by weight of the new iodopropargyl ether.

5. Use of microbicidal agents according to Claims 3 and 4, for the preservation of industrial materials.

6. Use according to Claim 5, for preserving wood against fungi which discolour and destroy wood.

**Revendications**

1. Ethers d'iodopropargyle de formule

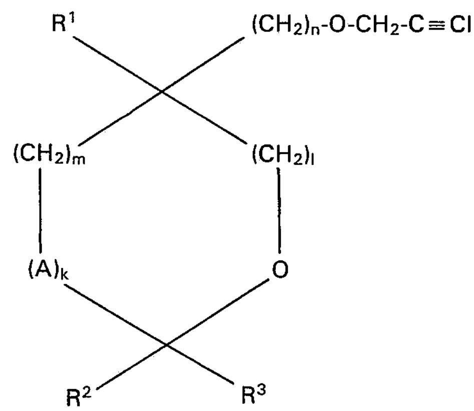

dans laquelle

A représente l'oxygène ou un groupe méthylène,

$R^1$ désigne l'hydrogène ou un groupe alkyle en $C_1$ à $C_6$,

$R^2$ et $R^3$ sont identiques ou différents et représentent l'hydrogène, un groupe alkyle en $C_1$ à $C_6$, alcényle ou phényle éventuellement substitué par un halogène, ou formant ensemble et conjointement avec l'atome de carbone qui les porte, un noyau carbocyclique de 4 à 7 atomes de carbone,

l et m ont la valeur 0, 1 ou 2,

n est un nombre entier de 0 à 4, sous réserve que lorsque 1 = 0, n ait la valeur 1, 2, 3 ou 4 et

k a la valeur 0 ou 1.

2. Procédé de production des éthers d'iodopropargyle nouveaux de formule

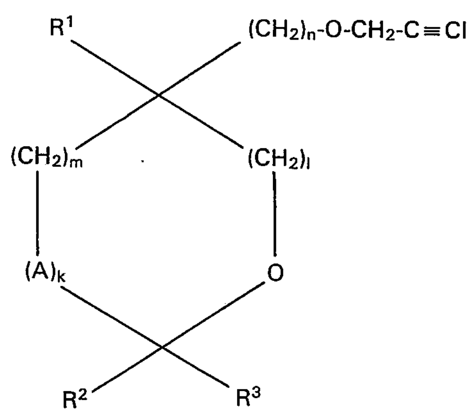

dans laquelle

A représente l'oxygène ou un groupe méthylène,

$R^1$ désigne l'hydrogène ou un groupe alkyle en $C_1$ à $C_6$,

$R^2$ et $R^3$ sont identiques ou différents et représentent l'hydrogène, un groupe alkyle en $C_1$ à $C_6$, alcényle ou phényle éventuellement substitué par un halogène, ou forment ensemble et conjointement avec l'atome de carbone qui les porte , un noyau carbocyclique de 4 à 7 atomes de carbone,

l et m ont la valeur 0, 1 ou 2,

n est un nombre entier de 0 à 4, sous réserve que lorsque 1 = 0, n ait la valeur 1, 2, 3 ou 4 et

k a la valeur 0 ou 1,

caractérisé en ce qu'on fait réagir avec des agents d'iodation, en présence de solvants et/ou de diluants et en présence de bases, à des températures de –10 à 30°C, des éthers de propargyle de formule

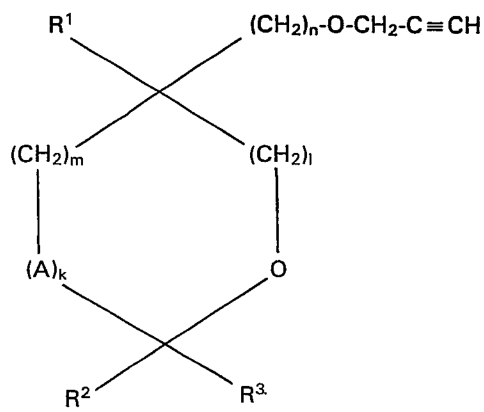

dans laquelle

A, $R^1$, $R^2$, $R^3$, l, m, n et k ont la définition indiquée ci-dessus.

3. Composition microbicide, contenant les éthers d'iodopropargyle nouveaux de formule

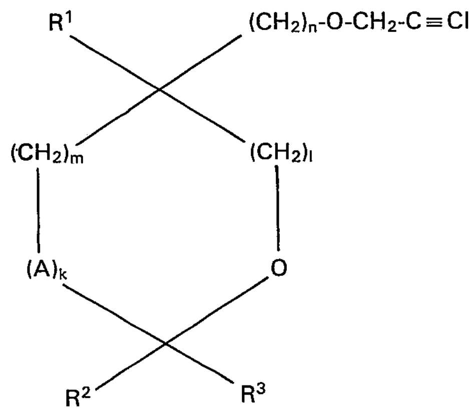

dans laquelle

A représente l'oxygène ou un groupe méthylène,

$R^1$ désigne l'hydrogène ou un groupe alkyle en $C_1$ à $C_6$,

$R^2$ et $R^3$ sont identiques ou différents et représentent l'hydrogène, un groupe alkyle en $C_1$ à $C_6$, alcényle ou phényle éventuellement substitué par un halogène, ou forment ensemble et conjointement avec l'atome de carbone qui les porte, un noyau carbocyclique de 4 à 7 atomes de carbone,

l et m ont la valeur 0, 1 ou 2,

n est un nombre entier de 0 à 4, sous réserve que lorsque 1 = 0, n ait la valeur 1, 2, 3 ou 4 et

k a la valeur 0 ou 1.

4. Composition microbicide suivant la revendication 3, contenant 1 à 95% en poids de l'éther d'iodopropargyle nouveau.

5. Utilisation de compositions microbicides suivant les revendications 3 et 4 pour la protection de matériaux industiels.

6. Utilisation suivant la revendication 5, pour protéger le bois de champignons qui en modifient la couleur et qui le détruisent.